# EUROPÄISCHE PATENTSCHRIFT

(11) **EP 2 662 105 B1**
(45) Veröffentlichungstag und Bekanntmachung des Hinweises auf die Patenterteilung: **18.01.2017**
(21) Anmeldenummer: 12167219.0
(22) Anmeldetag: 09.05.2012
(51) Int. Cl.: A61M 16/00, A61M 15/00, A61M 11/02

(54) **Zerstäuber**
Atomiser
Pulvérisateur

(43) Veröffentlichungstag der Anmeldung: 13.11.2013
(73) Patentinhaber: Boehringer Ingelheim International GmbH, 55216 Ingelheim am Rhein (DE)
(72) Erfinder: Winkler, Robert Gerhard, 55216 Ingelheim am Rhein (DE); Jung, Andree, 55216 Ingelheim am Rhein (DE); Wachtel, Herbert, 55216 Ingelheim am Rhein (DE)
(74) Vertreter: Simon, Elke Anna Maria

(56) Entgegenhaltungen:
- EP-A1- 0 820 780
- EP-A1- 1 731 186
- WO-A1-02/056948
- WO-A1-2004/110536
- US-A- 4 171 000
- US-A1- 2005 121 025
- US-A1- 2009 293 873

## Beschreibung

Die vorliegende Erfindung betrifft Geräte zur Zerstäubung von medizinischen, Formulierungen, bei der die Zerstäubung der Formulierung durch Treibmittel unterstützt wird. Insbesondere betrifft die Erfindung Geräte, die als Inhalatoren zur Verabreichung von pulverförmige Formulierungen verwendet werden können.

Der Einsatz von Medikamenten, die mittels Inhalatoren verabreicht werden, zielt insbesondere auf Wechselwirkungen mit speziellen Bereichen im Atmungssystem eines Patienten ab. Diese Bereiche beinhalten die Nasengänge, den Rachenraum und verschiedene Stellen innerhalb der Lunge, wie die Bronchien, Bronchiolen und Alveolen. Die Möglichkeit, Medikamente in einem dieser Zielbereiche zu verabreichen, hängt unter anderem von den aerodynamischen Größen der jeweiligen Partikel oder Tröpfchen ab, die bei Anwendung des Inhalators eingeatmet werden. Derzeit geht man davon aus, dass Partikel mit aerodynamischen Durchmessern zwischen 2 und etwa 5 Mikrometern gut den Bronchien und Bronchiolen zugeführt werden können. Kleinere Partikel können potentiell in die Alveolen vordringen. Partikel, deren aerodynamische Durchmesser größer 6 Mikrometer und insbesondere größer als 10 Mikrometer sind, eignen sich typischerweise zur Ablagerung im Rachenraum und in den Nasengängen.

Bei Inhalatoren, mit denen ein Medikament in die Lunge eingebracht werden soll, ist es in der Regel wünschenswert, wenn ein hoher Anteil des verabreichten Medikaments insbesondere hinsichtlich der Teilchengröße inhalierbar ist und eine hohe Ablagerungsrate des Medikaments in der Lunge selbst erzielt werden kann. Dies hängt von mehreren Faktoren ab, wie insbesondere den Eigenschaften einer mit dem Inhalator erzeugten Sprühwolke. Diese Eigenschaften sind beispielsweise die Geschwindigkeit der Wolke, die Größen der Partikel und ihre Größenverteilung, der Anteil kleiner Partikel, die Bestandteile des Gases usw.. In der vorliegenden Erfindung weist die Sprühwolke vorzugsweise einen hohen Anteil von Partikeln mit Durchmessern von 6 Mikrometern oder kleiner, bevorzugt von kleiner 5 Mikrometern auf.

Die vorliegende Erfindung bezieht sich auf die Zerstäubung von medizinischen Formulierungen. Unter dem Begriff "medizinische Formulierung" oder "Arzneimittelformulierung" sind bei der vorliegenden Erfindung über Medikamente hinaus auch Therapeutica oder dergleichen, insbesondere also jede Art von Mitteln zur Inhalation oder sonstigen Anwendung zu verstehen. Der Begriff "Formulierung" bezieht sich hier insbesondere auf Pulver, kann aber auch Flüssigkeiten beinhalten. Dementsprechend können die Partikel sowohl fest als auch flüssig sein. Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen.

Die folgende Beschreibung wird sich auf die Verabreichung von pulverförmigen Formulierungen konzentrieren, die Erfindung ist aber auch auf die Verabreichung von flüssigen Formulierungen anwendbar.

Insbesondere bezieht sich die vorliegende Erfindung auf Inhalatoren zur Einbringung von trockenem Pulver in die Lunge. Viele Pulverinhalatoren sind bereits im Handel oder anderweitig bekannt. Man unterscheidet zwischen zwei Arten von Inhalatoren: aktiven und passiven. In passiven Inhalatoren wird allein der Einatemsog des Anwenders bzw. des Patienten für die Deagglomeration des Pulvers und für den Transport des Pulvers in die Lunge verwendet. Der Transport und die Deagglomeration des Pulvers sind daher abhängig von der Flussrate, mit welcher der Patient Luft durch das Gerät eingeatmet. Dadurch ist der inhalierbare Anteil bzw. der Teil des Pulvers, das tatsächlich die Lunge erreicht, stark vom Einatemverhalten des Patienten abhängig und variiert von Patient zu Patient. Aktive Inhalatoren beinhalten im Gegensatz zu passiven Inhalatoren eine zusätzliche Energiequelle, welche die Deagglomeration und den Transport des Pulvers unterstützt. Das Pulver wird mit solchen Geräten aktiv zerstäubt.

Aktive Inhalatoren mit einem langjährigen hohen Marktanteil sind insbesondere konventionelle Treibmittel-getriebene Dosieraerosole (im Folgenden häufig mit der im Englischen üblichen Abkürzung MDI für "metered dose inhaler" bezeichnet). In einem MDI liegt das Medikament als Suspension oder Lösung in einem Treibmittel vor. Das Treibmittel befindet sich unter Überdruck in einem Behälter mit einem Dosierventil. Bei Aktivierung gibt das Dosierventil eine einzelne Einheit des Medikaments in Form eines Gasstroms aus. Für MDIs geeignete Treibmittel können Hydrofluorkarbonat oder andere Fluorkohlenwasserstoffe mit niedrigen Siedepunkten beinhalten. Bei konventionellen MDIs tritt das Treibmittel mit einer hohen Geschwindigkeit und teilweise flüssig aus dem Gerät aus. Dadurch wird ein großer Anteil des Medikaments in einer derart groben Form abgegeben, dass er sich direkt im Mund-Rachenraum eines Patienten ablagert ohne in die Lunge transportiert werden zu können.

Die WO2004110536A1 zeigt ein Ausgabegerät für ein pulverförmiges Medikament, in der eine Aufnahme mit pulverförmigen Medikament und eine Treibmittelquelle derart in einem Gehäuse angeordnet sind, dass das Treibmittel über einen Einlass direkt auf das pulverförmige Medikament trifft. In der Aufnahme kann sich das dabei gebildete Aerosol ausdehnen, bevor es über eine Ausgabeöffnung aus dem Gerät abgegeben wird. Für das Gerät sind Wechseleinsätze mit kombinierten Pulver- und Treibmittel-Einsätzen vorgesehen. Als mögliche Treibmittelquellen werden komprimierte Gase wie Kohlendioxid, Stickstoff oder Luft oder als konventionelle Treibmittel Fluorkohlenwasserstoffe wie HFA-134a oder HFC-227 angegeben. Im Rahmen von Versuchen für Klinikstudien werden in der Schrift WO2004110536A1 u.a. Zerstäubungsergebnisse zu Geräte-Konfigurationen mit 120 Milligramm mikronisiertem Wirkstoff gefüllten Aufnahmen und Stickstoff als Treibmittel mit Drucken von 6 bis 14 bar aufgeführt. Im Vergleich zu den geschilderten Versuchsdaten bringen handelsübliche (nicht kapselbasierte, meist passive) Pulverinhalatoren in der Regel lediglich Pulvereinheiten in der Größenordnung von 6 bis 400 Mikrogramm pro Anwendung aus, bzw. es ist auch ein kapselbasierter Pulverinhalator bekannt, in dem die Kapsel 28 Milligramm aufnimmt. Inhalatoren zur Ausbringung größerer Formulierungsmengen sind in diesem Zusammenhang - soweit den Erfindern bekannt - nicht im Markt erhältlich.
Die Schrift US4534345 zeigt beispielsweise einen aktiven Inhalator, der einen Treibmittel-Behälter, eine Vorratskammer mit pharmazeutisch aktiver Substanz in fester, mikronisierter Form und eine Dosis-Lade-Vorrichtung mit einer perforierten Membran beinhaltet. In einer ersten Position werden Perforationen in der Membran mit der Substanz befüllt, in einer zweiten werden die Perforationen in einen Kanal für das Treibmittel eingeschoben. Bei Betätigung einer zugehörigen Vorrichtung wird das Treibmittel aus seinem Behältnis in den Kanal abgegeben und trägt die Substanz durch eine Düse des Inhalators aus.
Die Schrift WO02/056948 A1 zeigt einen Trockepulverinhalator, in dem ein Gasstrom auf Pulver trifft, mit diesem eine Dispersion bildet, die zur effektiven Deagglomeration der Pulverpartikel auf einen Impaktor gelenkt wird. Optional fließt die Dispersion zuvor durch eine Engstelle, die beispielsweise durch den Auslass einer Wirbelkammer oder durch eine Röhre mit einem Innendurchmesser von 0,1 mm bis 4 mm gebildet wird. Für den Gasstrom wird Druckgas, z.B. Treibgas aus der Verdampfung von flüssigem Treibmittel oder in einem Behälter bevorratetes komprimiertes Gas wie Kohlendioxid, Stickstoff oder Luft, verwendet. Bei der Verwendung von flüssigem Treibmittel wird dieses mittels eines Ventils in eine Expansionskammer zur Verdampfung abgemessen.

Die Schrift US 2009/293873 zeigt einen Trockenpulverinhalator mit einem Wärmetauscher der vor der Pulverkavität angeordnet ist. Somit trifft der Gasstrom erst nach der Durchströmung des Wärmetauschers auf die Pulverpartikel in der Pulverkavität.

Inhalatoren für trockenes Pulver werden unterteilt in Geräte für die Verabreichung einzelner Dosiseinheiten und in Mehrdosis-Geräte.
Mehrdosis-Geräte werden des weiteren unterteilt in Geräte mit vorabgemessenen und einzeln gelagerten Dosis-Einheiten (hier wird die übliche Abkürzung "pmDPl" verwendet, welche der entsprechenden englische Bezeichnung "pre-metered Dry Powder Inhaler" entstammt) und in solche Geräte, in denen jede Pulver-Einheit innerhalb des Geräts aus einem Reservoir mittels einer Abmessvorrichtung abgemessen wird.
Die Verwendung von pmDPIs hat den Vorteil, dass die einzelnen Dosis-Einheiten unter überwachten Fabrik-Bedingungen abgemessen werden und das Pulver dabei vergleichsweise einfach von Umgebungsluft und anderen äußeren Einflüssen geschützt werden kann. Bei vielen Anwendungen wird eine Formulierung in Form einer Mischung aus einem Wirkstoffs und einer Trägersubstanz wie Laktose eingesetzt. Die Laktose und/oder der Wirkstoff bzw. die Wirkstoffe neigen zur Absorption von Flüssigkeit aus der Umgebungsluft, wodurch es zur Verklumpung des Pulvers und zu Schwierigkeiten bei der De-Agglomeration bzw. der Zerstäubung und beim Transport des Pulvers in die Lunge kommt.
Die Schrift DE4106379A1 zeigt ein passives Inhalationsgerät, in dem sich das Pulver in vordosierten inhalierbaren Pulvermengen in Taschen eines biegsamen streifenförmigen Trägers befindet. Dieser Träger besteht aus einer die Taschen bildenden Trägerbahn und einer die Taschen verschließenden Abdeckbahn. Das Inhalationsgerät weist neben einer Aufnahme für den Pulverträger eine Öffnungsstation mit einer Abziehvorrichtung auf, die zum Öffnen der Taschen die Abdeckbahn und die Trägerbahn voneinander abziehen. Durch einen Pulverauslass kann Pulver aus der geöffneten Tasche angesaugt werden.

Die vorliegende Erfindung bezieht sich insbesondere auf ein so genanntes aktives Mehrdosis-Gerät zur Abgabe eines Wirkstoffs oder einer Wirkstoff beinhaltenden Formulierung zur Inhalation.
Insbesondere bezieht sich die Erfindung auf solche pmDPIs, in denen Druckgas - vorzugsweise in Form von Druckluft - und/oder Treibmittel, wie vorzugsweise HFA-Gases wie Typ 134a, bei der Zerstäubung des Pulvers eingesetzt werden. Die Schrift EP1992381A1 offenbart einen aktiven pmDPI mit einer ringförmigen schrittweise drehbaren Vorratsvorrichtung mit mehrfachen Einschüben, wobei jeder Einschub eine einzelne Dosis einer medizinischen Formulierung in einer Vorratskammer und eine Düse beinhaltet. Die Einschübe befinden sich in getrennten und versiegelten Kavitäten, die individuell für die Ausbringung der einzelnen Dosis geöffnet werden. Zur Ausbringung der Dosis wird der jeweilige Einschub über ein Verbindungselement mit einer Luftpumpe mit Faltenbalg verbunden.
Die Schrift WO2009083244A2 zeigt einen aktiven pmDPI, in dem sich die einzelnen Dosen der medizinischen Formulierung in Vorratseinheiten in einem länglichen Träger befinden. Außer einer Vorratskammer mit Formulierung umfasst jede Vorratseinheit eine Düse zur individuellen Abgabe der jeweiligen Dosis. In einer Ausführungsform werden die Düsen über das Abziehen eines Deckstreifens nach einander freigelegt. Zum Ausbringen der Formulierung aus den einzelnen Vorratseinheiten wird darüber hinaus die jeweilige zugehörige Vorratskammer mit einem Anstechelement angestochen, durch das Druckgas in die Vorratskammer geleitet wird, wodurch die Formulierung mitgerissen wird. Das Druckgas wird durch eine Luftpumpe oder alternativ aus einem Behälter mit verflüssigtem Gas bereitgestellt. In einer Ausführungsform wird die Ausbringung der jeweiligen Dosis mittels Druckluft über eine Atemzugserkennung ausgelöst.

Der vorliegenden Erfindung liegt die Aufgabe zugrunde, ein gegenüber dem Stand der Technik verbessertes Gerät zur Zerstäubung von vorzugsweise pulverförmigen medizinischen Formulierungen zur Inhalation anzugeben. Insbesondere sollte ein Gerät - vorzugsweise ein kompaktes Handgerät - angegeben werden, bei dem vergleichsweise große abgemessene Substanzmengen - insbesondere größer 1 Milligramm - zur Inhalation ausgebracht werden können bzw. mit dem große inhalierbare Dosismengen der medizinischen Formulierung bereitgestellt werden können. Besonders bevorzugt sollte bei der Ausgestaltung des Geräts als Inhalator der lungengängige Anteil einer als Aerosol mit diesem Gerät ausgebrachten Formulierungsmenge nur in einem geringen Maß oder gar nicht vom Einatemverhalten des Anwenders, d.h. des Patienten sein. Darüber hinaus sollte das Gerät insbesondere hinsichtlich des Aspekts der fehlerfreien Anwendung durch einen Anwender, insbesondere bzgl. der Aspekte Laden einer Dosiseinheit im Gerät und/oder Koordination des Einatemverhaltens mit der Zerstäubung vorteilhaft gestaltet sein.

Die genannte Aufgabe wird erfindungsgemäß durch ein Gerät zur Zerstäubung von medizinischen Formulierungen, gemäß Anspruch 1 gelost, wobei im Gerät die Zerstäubung durch ein Treibmittel unterstützt wird, das einer Kavität zugeführt wird, in der sich eine abgemessene Menge der Formulierung befindet, und das vor dieser Zuführung durch einen Verdampfer oder Wärmetauscher geleitet wird. Der mit Formulierung beladener Treibmittelstrom wird von der Kavität aus in eine Düse geleitet, wobei die Düse einen im Wesentlichen geradlinigen Düsenkanal aufweist.
Weitere Merkmale der vorliegenden Erfindung ergeben sich aus den Ansprüchen.

Vorteilhafte Weiterbildungen werden im Folgenden und im Detail anhand der Figuren beschrieben.

Ein Merkmal der vorliegenden Erfindung ist, dass der vorzugsweise aus Metall gefertigte Verdampfer einen Hohlraum mit einem Einlass und einen Auslass für das Treibmittel aufweist. An den Einlass wird vorzugsweise eine Kartusche mit dem Treibmittel angeschlossen, wobei diese Kartusche vorzugsweise ein Dosierventil an der Anschlussstelle aufweist.
Der Verdampfer bewirkt, dass das Treibmittel, das z.B. in einer Vorratskartusche flüssig vorliegt, vollständig oder nahezu vollständig in den gasförmigen Zustand überführt wird, bevor es der Kavität mit der Formulierung zugeführt wird. Das in einer konventionellen Kartusche unter Druck flüssig gehaltene Treibmittel verdampft unter Normaldruck in der Regel bei negativen Temperaturen auf der Celsius-Temperatur-Skala. Wird es in den Hohlraum des Verdampfers eingeleitet, kann es dort expandieren und wechselt vom flüssigen in den gasförmigen Zustand. Insbesondere bei der Verwendung pulverförmiger Formulierungen wird dadurch verhindert, dass flüssiges Treibmittel das Pulver verklumpt und die Zerstäubung des Pulvers dadurch beeinträchtigt wird.

Ein weiteres Merkmal der vorliegenden Erfindung ist, dass der Verdampfer in seinem Innern Wärmetausch-Elemente beinhaltet. Die Wärmetausch-Elemente unterstützen bzw. beschleunigen die Verdampfung des flüssigen Treibmittels im Verdampfer, in dem sie Wärme an das an ihnen entlang strömende Treibmittel abgeben. Diese Wärmetausch-Elemente sind vorzugsweise aus Metall und weisen eine relativ große Oberfläche auf, was ebenfalls den Verdampfungseffekt begünstigt. Vorzugsweise haben die Wärmetausch-Elemente hierzu die Form von Kugeln und/oder Drähten.

Ein weiteres Merkmal der vorliegenden Erfindung ist, dass im Innern des Verdampfers die Bauteile des Verdampfers derart gestaltet sind, das sie für das durch den Verdampfer strömende Treibmittel einen möglichst geringen Strömungswiderstand darstellen. Dadurch kann nahezu die volle Geschwindigkeit der Treibgasströmung für die Pulverzerstäubung genutzt werden. Zur Reduzierung des Strömungswiderstandes tragen bei:
- die rotationssymmetrische Gestalt des Verdampfers,
- konusförmige Übergänge im Einlass- und Auslassbereich des Hohlraums des Verdampfers,
- Kugelstruktur der Wärmetausch-Elemente im Verdampfer und/oder
- eine derartige Bemessung der Wärmetausch-Elemente, dass es durch sie zu keiner Verlegung von Ein-oder Auslass des Verdampfers kommt und dass ihre Zwischenräume gut durchströmbar sind.

Ein weitere Merkmal der vorliegenden Erfindung ist, dass die Zuleitung von Treibmittel in die Kavität mit der Formulierung und die Achse eines vorzugsweise geradlinigen Düsenkanals an der gleichen Stelle, bevorzugt mittig, und unter dem gleichen Winkel relativ zum Boden der Kavität eintreffen. Bei dieser Anordnung erstreckt sich die Achse des Düsenkanals, durch den die Formulierung mittels Treibmittel aus der Düse des Geräts bzw. des Zerstäubers ausgebracht wird, gerade in der Richtung, in welche der Treibmittelstrom von der Pulverkavität aus zum größten Teil reflektiert wird. Auf diese Weise stößt der mit Formulierung behaftete Treibmittelstrom auf seinem Weg nach draußen nicht unnötig an die Wände der Kavität. Die Kavität wird besser entleert bzw. es kommt nicht oder kaum zu Ablagerungen von Formulierung neben dem Einlass des Düsenkanals. Ein Winkel von 45° ist hierbei für den Aufbau eines kompakten Zerstäubers vorteilhaft - beispielsweise wenn mehrere Kavitäten entlang eines kreisförmigen Radius nacheinander in die Position zur Ausbringung der Formulierung gebracht werden. Aber auch etwas geringere Winkel wie z.B. 30° können insbesondere bei länglichen Kavitäten vorteilhaft sein, da dann mehr Formulierung vom Treibmittel sozusagen durchstoßen werden muss, bevor der Treibmittelstrom reflektiert wird. Dieser Reflektionswinkel sollte an die optimale Gestaltung von Kavität und gegebenenfalls an einen Austauschmechanismus für mit Formulierung gefüllte Kavitäten angepasst werden.

Ein weiteres Merkmal der vorliegenden Erfindung ist, dass das Gerät im Falle einer Ausführungsform als Inhalator zur Ausbringung mehrerer einzeln abgemessenen Mengen der Formulierung dient. Hierzu befinden sich die einzelnen Mengen der Formulierung - z.B. einzelne Pulvereinheiten - bevorzugt in den Kavitäten eines Blisterbands. Die Kavitäten werden dann nacheinander z.B. durch eine Weiterbewegung des Blisterbands in eine Entnahmeposition im Treibgasstrom gebracht. Zuvor sind sie z.B. über einen Mechanismus, durch den die jeweilige Kavität beispielsweise angestochen wird oder durch den vorzugsweise eine die Kavität verschließenden Deckfolie abgezogen wird, geöffnet worden. Die Bevorratung der Kavitäten entlang eines Blisterbands hat den Vorteil, dass durch Rollen bzw. Wickeln des Bandes viele Kavitäten auf kleinem Raum bevorratet werden können. Besonders bevorzugt wird der Transport des Blisterbandes im Inneren des Geräts durch eine Bewegung außen am Gerät gesteuert, wie insbesondere das Öffnen oder Schließen einer Mundstückabdeckung.
Ein weiteres Merkmal der vorliegenden Erfindung ist, dass die Ausbringung der Formulierung aus der Kavität durch eine Düse erfolgt, deren Auslassöffnung in ein in Strömungsrichtung verlängertes Mundstück einmündet. Bevorzugt ist dabei der Überstand des Mundstücks im Vergleich zum Düsenauslass länger als der Düsenkanal in der Düse. Bevorzugt ragt das Mundstück 40 bis 120 Millimeter, besonders bevorzugt 40 bis 70 Millimeter über das Ende der Düse hinaus. Bei einem derart bemessenen Überstandsbereich ist einerseits der Anteil des lungengängigen Feinanteils des ausgebrachten Aerosols gegenüber Geräten mit kleineren Überständen erhöht und andererseits ist der Überstand noch nicht so groß, als das es zu einer übermäßigen Bildung von Ablagerungen innen im Mundstück kommt. Bei den größeren Überstandsbereichen - insbesondere bei Überständen von 70 bis 120 Millimeter - nimmt der Anteil der inhalierbaren Dosis weiter zu, es muss lediglich gegebenenfalls eine regelmäßige Reinigung des Mundstücks vorgesehen werden.
Zur Ausbildung einer für die Inhalation des gebildeten Aerosols günstigen Aerodynamik im Mundrohr weist das Mundstück des Weiteren geräteseitig (d.h. auf der Seite, die der Stelle entgegengesetzt liegt, an der im Falle eines Geräts zur Inhalation an Anwender seine Lippen ansetzen würde) mindestens eine, bevorzugt 1 bis 4 Einlassöffnung auf. Bevorzugt ist die Einlassöffnung am Mundstück so gestaltet, dass es zu einer Bypass-Luftströmung nahe dem Auslass der Düse kommt, die besonders bevorzugt den aus der Düse austretenden mit Formulierung beladenen Treibmittelstrom umhüllt. Es zeigt sich, dass die Aerodynamik weiterhin günstig beeinflusst wird, wenn der innere Durchmesser des Mundstücks an der Stelle des Düsenauslasses deutlich größer, insbesondere fünfmal, so groß wie der Duchmesser der Öffnung des Düsenauslasses ist.

Ein weiteres Merkmal der vorliegenden Erfindung ist, dass das Gerät im Falle einer Ausführungsform als Inhalator eine Atemzugstriggerung aufweist, welche die Zuführung von Treibmittel in den Verdampfer auslöst. Bevorzugt befindet sich ein Schaltelement der Atemzugstriggerung z.B. in Form eines Strömungssensors im Bereich der Einlassöffnungen des Mundstücks und/oder in einem an die Einlassöffnungen angeschlossenen Kanal.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
Fig. 1 einen schematischen Schnitt durch die Zerstäubungseinheit eines erfindungsgemäßen Geräts,
Fig. 2 einen erfindungsgemäßen Zerstäuber, der als Testvorrichtung zur Funktionsweise eines erfindungsgemäßen Inhalators aufgebaut wurde.
Fig. 3a einen schematischen Schnitt durch einen Teil der Treibmittelzuführungsvorrichtung in einem erfindungsgemäßen Zerstäuber und Fig. 3b einen schematischen Schnitt durch zwei Einzelbauteile aus der Treibmittelzuführung,
Fig. 4a einen schematischen Schnitt durch die Zerstäubungseinheit eines erfindungsgemäßen Geräts, wobei in Fig. 4b und ebenfalls in Schnittansicht in Fig. 4c eine in diesem Gerät eingesetzte Düse dargestellt ist, und
Fig. 5 einen erfindungsgemäßen Inhalator als Handgerät, wobei Fig. 5a den Inhalator von außen mit geschlossener Mundstückabdeckung, Fig. 5b in schematischer Schnittansicht und Fig. 5c von außen mit geöffnetem Mundstück zeigt.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

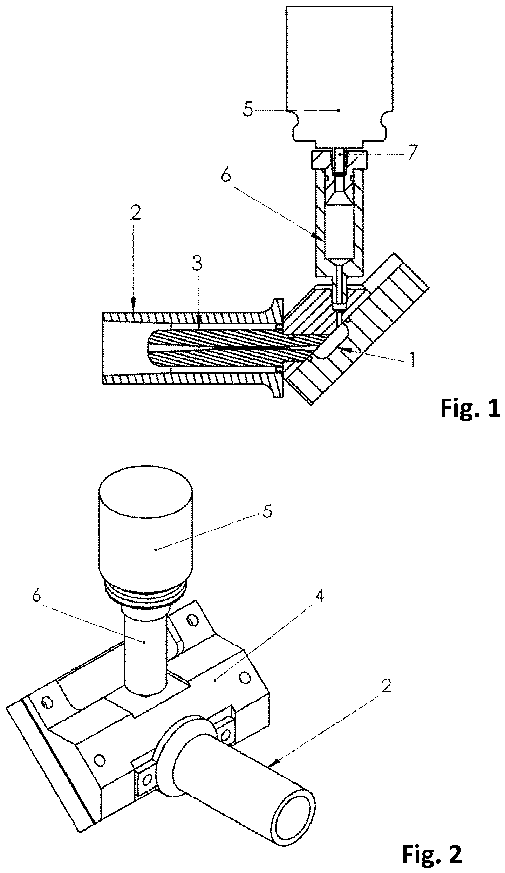

Fig. 1 zeigt in schematischer Schnittdarstellung die Funktionsweise eines erfindungsgemäßen Geräts, das insbesondere für die Zerstäubung von Pulvern geeignet ist. Der Aufbau des dargestellten Geräts bzw. Zerstäubers eignet sich sowohl für einen erfindungsgemäßen Inhalator als auch vor allem für eine Testvorrichtung zur Überprüfung der Funktionsweise einzelner jeweils austauschbar gehaltenen Komponenten des Inhalators. Eine solcher als Testvorrichtung gestalteter Zerstäuber ist in Fig. 2 abgebildet.
Bei der Zerstäubung von Pulvern wird hierbei Treibmittel aus einer Kartusche (5) direkt oder vorzugsweise nach vollständiger Durchführung durch einen Verdampfer (6) oder Wärmetauscher in eine Pulverkavität (1) geleitet, auf die eine Düse (3) aufgesetzt ist, die wiederum in ein Mundstück (2) einmündet. Bei der hier bevorzugten Verwendung eines Verdampfers (6), sollte dieser so im System eingebaut sein, dass ausgeschlossen ist, dass ein Teil des aus dem Ventil an der Kartusche (5) abgegebenen Treibmittels über einen Bypass am Verdampfer (6) vorbei in die Pulverkavität (1) gelangen kann. Das Treibmittel treibt das Pulver aus der Pulverkavität (1) durch die Düse (3) aus. Saugt gleichzeitig ein Anwender bzw. Patient am Mundstück (2) Luft an, so wird das Pulver beim Austritt aus der Düse (2) von der Einatemluft mitgerissen, die an der Düse vorbei durch das Mundstück (2) in die Lunge strömt. Das Pulver gelangt so durch den Atemsog des Patienten in dessen Lunge.
Sowohl die Testvorrichtung als auch ein gemäß gleicher Funktionsweise aufgebauter Inhalator wird vorzugsweise - analog zur Bedienung eines konventionellen Dosieraerosols (im Folgenden wird häufig mit der aus dem Englischen üblichen Abkürzung MDI bezeichnet) - so bedient, dass der Boden der Kartusche (1) nach oben zeigt, d.h. dass der Ventilstamm (7) der Kartusche (1) nach unten gerichtet ist. Das Treibmittel wird mittels eines zur Kartusche gehörenden Ventils (ebenfalls in Analogie zum konventionellen MDI) dosiert, wobei die Abgabe einer Einheit des Treibmittels beispielsweise über Druck auf den Boden der Kartusche (5) ausgelöst wird.

Bei der in Fig. 2 gezeigten Testvorrichtung besteht der Zerstäuber aus einzelnen Modulen. Die funktionsrelevanten Module sind hierbei insbesondere die Kartusche (5), der Verdampfer (6), die Düse (3), das Mundstück (2) und eines, das die Pulverkavität (1) bildet. Zusätzlich kann im Fall der Testvorrichtung noch ein Anschlusstück (4) hinzukommen, an dem andere Module insbesondere die Düse (3), die Pulverkavität (1) und der Verdampfer (6) oder die Kartusche (5) austauschbar befestigt und in fluidische Beziehung zueinander gebracht werden können.
Die Testvorrichtung bietet den Vorteil, dass die in die Testvorrichtung eingesetzten Module variiert werden können und die Auswirkung der jeweiligen Variation auf das Zerstäubungsverhalten bestimmt werden kann. Bevorzugt wird außerdem ein System zur Strömungserzeugung - z.B. eine laborübliche Steuerwand mit konventioneller Pumpe - an die Testvorrichtung angeschlossen, so dass verschiedene Saugstärken am Mundstück (2) nachgestellt werden können. Die Zerstäubung des jeweils in der Testvorrichtung eingesetzten Pulvers kann mit den gleichen Methoden wie die Zerstäubung aus Inhalatoren untersucht werden, insbesondere mittels Laserbeugungsmethoden, Hochgeschwindigkeitskameras und Kaskadenimpaktoren. Die in dieser Schrift gezeigten Messergebnisse wurden mit einem gemäß den Vorgaben des Europäischen Arzneibuchs (Version 6.0) gestalteten Kaskadenimpaktor zur Bestimmung des Feinanteils der ausgebrachten Dosis ermittelt (wobei zusätzlich bei verschiedenen Flussraten gemessen wurde). Dieser Feinanteil wird bezogen auf Partikelgrößen mit Durchmessern kleiner als 5 Mikrometern und wird im Folgenden zuweilen auch mit der Abkürzung FPD bezeichnet (hergeleitet aus der oft angewandten englischen Bezeichnung "Fine Particle Dose"). Bei den Messungen mit dem hierzu eingesetzten Kaskadenimpaktor wurden die Wirkstoff-Ablagerungen auf den einzelnen, die Kaskadenstufen bildenden Schalen und der Anschluss des Kaskadenimpaktors jeweils pro Messung individuell gewaschen und die sich ergebenden Lösungen wurden mittels Hochdruck-Flüssigkeits-Chromatographie untersucht. In den einzelnen Kaskadenstufen werden während der Messung unterschiedliche Größenklassen der zerstäubten Partikel gesammelt, so dass mittels der Chromatographie der Lösung aus der jeweiligen Kaskadenstufe die jeweiligen Anteile der Dosis pro Größenklasse bestimmt werden können.
Die Testvorrichtung wird bevorzugt für die Überprüfung der Zerstäubung von pulverförmigen medizinischen Formulierungen eingesetzt. Bevorzugte Formulierungen bestehen aus strahlgemahlenem und gesiebtem Wirkstoff, der mit Laktose gemischt wird. Beliebige Mischungsverhältnisse bis hin zu reinem Wirkstoff können mit der Testvorrichtung überprüft werden.
Für die meisten Messungen wurde in der Testvorrichtung eine Pulverkavität (1) mit einem Fassungsvermögen von 0,2 Millilitern verwendet, in die meist 50 Milligramm einer auf Laktose basierten Formulierung mit 32,5% einer aktiven pharmazeutischen Substanz (ein in der Entwicklung befindlicher Wirkstoff) eingefüllt wurden. Für das hier beschriebenen Zerstäubungskonzept können jedoch auch Pulverkavitäten (1) mit Pulvermengen von bis zu 100 Milligramm befüllt werden - getestet wurden beispielsweise 21 Milligramm, 60 Milligramm, 70 Milligramm und 75 Milligramm (einige davon mit einer Formulierung, die 98% Fenoterol-HBr beinhaltet, da sich dieser Wirkstoff gut für Extremalprüfungen der Zerstäubung von Inhalatoren eignet). Die Größe der Pulverkavität (1) kann an die Menge des aufzunehmenden Pulvers angepasst werden. Auch Pulvermengen bis hin zu 250 Milligramm sind möglich bei entsprechenden Anpassungen der Pulverkavität (1) und der Treibmittelzufuhr (hin zu größeren Drücken und umfangreicheren Treibmittelstößen) möglich.
Die Ausbringung solch im Vergleich zu handelsüblichen Pulverinhalatoren hoher Pulvermengen wird in diesem Fall durch die Verwendung von Treibmitteln ermöglicht. Bevorzugt werden hierbei Hydrofluoralkane - so genannte HFAs - verwendet. Besonders bevorzugt wird hier als Treibmittel HFA R134a (Norfluran oder 1,1,1-Trifluorethan bzw. 1,1,1,2-Tetrafluorethan) in der Kartusche (1) eingesetzt, wobei dass zur Kartusche gehörende Dosierventil 100 Mikroliter pro Aktivierung abgibt. Bei kleineren Formulierungsmengen können jedoch auch kleinere Treibmitteleinheiten verwendet werden, wie beispielsweise 50 Mikroliter Treibmittel bei 21 Milligramm Pulvermenge. Aber auch andere Treibmittel wie andere Hydrofluoralkane wie beispielsweise HFA 227ea (Apafluran oder 1,1,1,2,3,3,3-Heptafluoropropan), flüssiger Stickstoff oder konventionelle Treibmittel wie Halogenkohlenwasserstoffe können bei diesem Prinzip eingesetzt werden. In der Kartusche (5) liegt das Treibmittel - wie auch in handelüblichen Dosieraerosolen (MDIs) - in verflüssigter Form vor.

Die in Fig. 2 gezeigte Testvorrichtung wird bei einer Ausrichtung der Kartusche (5) senkrecht nach oben, mit nach unten gerichteten Ventilstamm (7) verwendet. Wenn beispielsweise durch Drücken auf den nach oben gerichteten Boden der Kartusche Treibmittel freigesetzt wird, fließt das Treibmittel zunächst in den Verdampfer (6) und von da aus als Treibgas über eine Zuleitung im Anschlussstück (4) in die Pulverkavität (1). Das Treibgas treibt dann das Pulver der Pulverkavität (1) durch die Düse (3) aus. Bevorzugt wird hierbei das Treibmittel HFA R 134A mit einem Druck von etwa 6 bar bei Raumtemperatur verwendet, mit dem vorteilhafterweise im System größere Flussgeschwindigkeiten als mit konventionellen Fluorkohlenwasserstoffen erzielt werden, die abgefüllt lediglich Drücke im Bereich von 2 bis 4 bar aufweisen.
Der Verdampfer (6) bewirkt, dass das mit dem Dosierventil abgegebene, abgemessene Treibmittel, das in der Kartusche (5) flüssig vorlag, komplett in den gasförmigen Zustand überführt wird, bevor es der Pulverkavität (1) zugeführt wird. Das Treibmittel wird dabei gewissermaßen getrocknet. Dadurch wird verhindert, dass flüssiges Treibmittel das Pulver verklumpt. Verklumptes Pulver lässt sich wesentlich schlechter dispergieren und die Lungengängigkeit der bei der Zerstäubung erzeugten Aerosol-Partikel verschlechtert sich. Videoaufnahmen mit einer Hochgeschwindigkeitskamera, mit der durch einen transparenten Boden der Pulverkavität (1) das Innere der Pulverkavität (1) während der Zuleitung von Treibmittel beobachtet wurde, belegen, dass es ohne die Verwendung eines Verdampfers (6) zu besagter Verklumpung kommt, während bei Verwendung des im Folgenden im Detail beschriebenen Verdampfers keine Agglomerate oder Verklumpungen mehr zu erkennen waren.

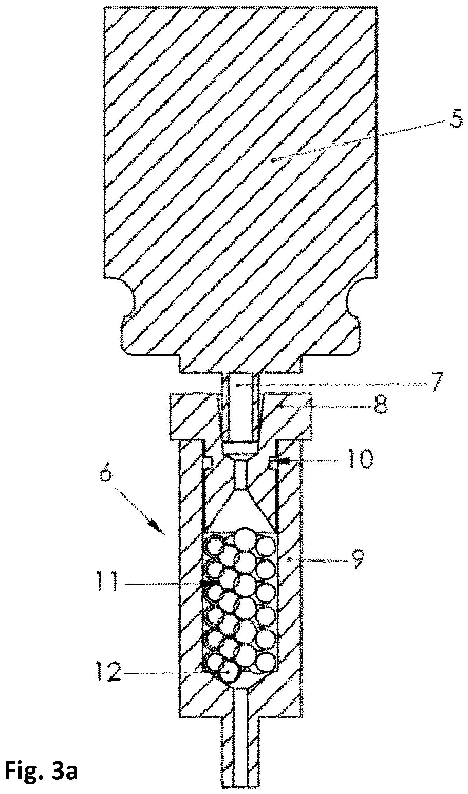

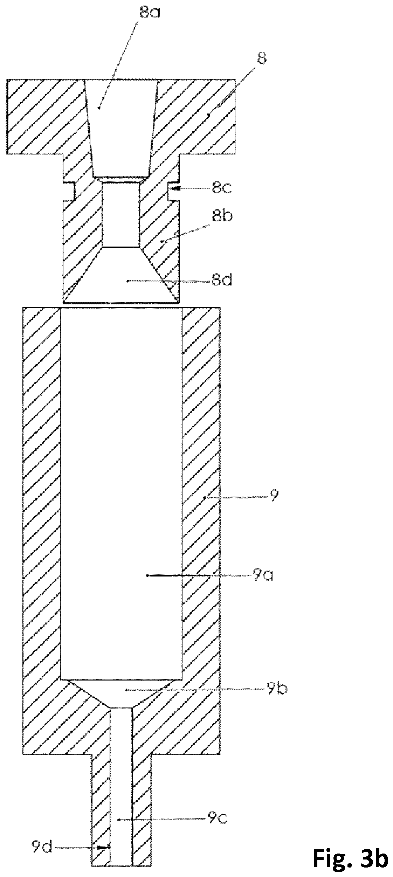

Fig. 3 zeigt einen möglichen Aufbau eines solchen Verdampfers (6). In der gezeigten Ausführungsform für den Einsatz in einer Testvorrichtung ist der Einlass des Verdampfers (6) so gestaltet, dass er einerseits eine Aufnahme (8a) für einen handelsüblichen Ventilstamm (7) einer Kartusche (5) aufweist und andererseits der Auslass (9c) so gestaltet ist, dass das Äußere des Verdampfers (6) an dieser Stelle gerade die Form eines Stammes (9d) mit den gleichen Abmessungen wie bei einem handelsüblichen Ventilstamm aufweist, beispielsweise einen Durchmesser von 2 Millimetern. Dementsprechend weist auch das Anschlussstück (4) eine Ventilstammaufnahme auf, an der für Versuchszwecke wie hier gezeigt der Verdampfer (6) oder wahlweise direkt die Kartusche (5) angeschlossen werden kann.
Der dargestellte, vorzugsweise rotationssymmetrisch gestaltete Verdampfer (6) beinhaltet einen Körper (9) mit einem vorzugsweise zylinderförmigen Hohlraum (9a). Der Boden des Hohlraums ist vorzugsweise als Trichter (9b) gestaltet, von dessen Mitte der Auslass (9c) als Kanal abgeht. Verschlossen wird der Hohlraum (9a) des Verdampfers durch einen Deckel (8), dessen Flansch (8b) in den oberen Bereich des Hohlraums (9a) eingetaucht wird. Vorzugsweise werden der Körper (9) und der Deckel (8) des Verdampfers (6) mittels einer Dichtung (10) gegeneinander abgedichtet. Diese Dichtung (10) ist beispielsweise - im Falle des rotationssymmetrischen Verdampfers (6) - ein ringförmiges Bauteil aus einem elastomeren Material. Sie wird beispielsweise in eine Sicke (8c) am Flansch (8b) des Deckels (8) eingelegt. Der Deckel weist eine Ventilstammaufnahme (8a) passend zur Aufnahme eines Ventilstamms einer handelsüblichen Kartusche (5) und eine Durchführung auf, die sich bevorzugt über einen Innenkonus (8d) zum Hohlraum (9a) hin öffnet.
Die Wirkung des Verdampfers (6) basiert auf Wärmetausch-Mechanismen. Der Verdampfer (6) selbst besteht vorzugsweise aus Metall und der Hohlraum ist bevorzugt ebenfalls mit vorzugsweise metallenen Bauteilen gefüllt, die im Folgenden als Wärmetausch-Elemente bezeichnet werden. Das in der Kartusche (5) unter Druck flüssig gehaltene Treibmittel verdampft unter Normaldruck - im Ausführungsbeispiel bei etwa -25°Celsius. Kann es also im Hohlraum des Verdampfers (6) expandieren, so wechselt es vom flüssigen in den gasförmigen Zustand. Die Wärmetausch-Elemente unterstützen bzw. beschleunigen diese Verdampfung, in dem sie Wärme an das an ihnen entlang strömende Treibmittel abgeben. Die Wärmetausch-Elemente haben hierzu bevorzugt eine möglichst große Oberfläche. Hierdurch wird die gesamte innere Oberfläche des Verdampfers erhöht und das Totvolumen, d.h. der freie Raum innerhalb des Verdampfers, verkleinert. Als Material werden für alle Bauteile des Verdampfers Metalle bevorzugt, da sie eine hohe Wärmeleitfähigkeit aufweisen und dadurch die Verdampfung des auf sie auftreffenden Treibmittels begünstigen. Möglich ist die Verwendung der meisten bei Raumtemperatur fester Metalle, wobei Edelstähle besonders bevorzugt sind, da sie zu den meisten Treibmitteln insofern eine gute Kompatibilität aufweisen, als dass keine Bestandteile des Metalls vom Treibmittel aufgenommen und der Zerstäubung zugeführt werden. Edelmetalle wie Silber und Gold wären ebenfalls gut geeignet, scheiden aber aus Kostengründen meistens aus. Sowohl für den Körper (9) des Verdampfers (6) als auch für Wärmetausch-Elemente in seinem Hohlraum (9a) werden hier konkret Aluminium, Edelstahl oder Kupfer als Materialien bevorzugt. In einer bei Versuchen verwendeten Ausführungsform ist der Körper (9) des Verdampfers (6) aus Aluminium, und der Hohlraum (9a) ist mit Kugeln (11) aus Edelstahl gefüllt. Je nach Verdampfungsverhalten des gewählten Treibmittels kann es gegebenenfalls ausreichen, wenn lediglich die Wärmetausch-Elemente aus Metall sind und der Körper (9) selbst aus Kunststoff ist. Dies bietet Kostenvorteile bei der Produktion.
Damit keine der Kugeln (10) den Auslass (9c) im Boden des Trichters (9b) verlegt, liegt beim konkreten Ausführungsbeispiel im Trichter (9b) ein gewundener Draht (12) - im konkreten Beispiel aus Kupfer -, der die Kugeln (10) vom Auslass (9c) fernhält.
Wahlweise können die Metallkugeln auch ganz durch einen langen, dünnen gewickelten Metalldraht im Hohlraum (9a) ausgetauscht werden. Im Ausführungsbeispiel haben die Kugeln (10) einen Durchmesser von 2 Millimetern, und dem steht ein Auslass (9c) mit einem Durchmesser von 1 Millimeter gegenüber.
Insgesamt ist die Gestalt bzw. die Anordnung der Bauteile so gewählt, dass der Verdampfer (6) einerseits zwar eine möglichst große innere Oberfläche für eine effiziente Verdampfung des Treibmittels aufweist, andererseits die Füllung mit Wärmetausch-Elementen hinreichend viele kleine freien Querschnitte aufweist, so dass sein Strömungswiderstand nicht so hoch wird, d.h. dass er das durch ihn durch geleitete Treibmittel nicht zu sehr verlangsamt. In diesem Zusammenhang wird hier bezogen auf Luft ein Strömungswiderstand von um die 46000 √N*s/m⁴ ± 1% bevorzugt (für Treibmittel sind noch geringere Strömungswiderstände als Luft zu erwarten). Dies entspricht einem zustande kommenden Fluss von 10 Liter pro Minute bei einem Druckabfall von 6 Kilopascal (ein geringerer Fluss als 5 Liter pro Minute bei einem Druckabfall von 6 Kilopascal würde beispielsweise einen ungünstigeren Strömungswiderstand darstellen, das Treibgas würde auf seinem Weg durch den Verdampfer dann deutlich abgebremst). Der Strömungswiderstand des Verdampfers wird durch seine Geometrie und durch die Größe und Form der in ihm enthaltenen Wämetausch-Elemente beeinflusst. Bei der hier bevorzugten Auslegung ermöglichen die Zwischenräume zwischen den Kugeln eine gute Durchströmbarkeit des Verdampfers, die Konturen in Übergangsbereichen sind durch die Verwendung von Konus-Strukturen strömungstechnisch günstig und die Kugelform der Wärmetausch-Elemente trägt ebenfalls dazu bei, dass sich keine oder nur geringe Turbulenzen in der Strömung bilden. Dadurch kommt es im Verdampfer nur zu einer geringen Druckabsenkung des Treibmittels, der Gasstrom wird also nur wenig verlangsamt. Dies ist in sofern von Vorteil als dass eine hohe Geschwindigkeit des Treibmittels wesentlich für eine gute Dispersion des Pulvers ist.
Die Geschwindigkeit des Treibmittels bei Eintritt in die Pulverkavität (1) kann durch die Größe der Durchmesser der Zuführungskanäle beeinflusst werden. Im Fall der Testvorrichtung können verschiedene Anschlussstücke (4) mit unterschiedlich breiten Zuführkanälen getestet werden. Bei den hier zugrunde liegenden Messergebnissen wurden Kanaldurchmesser im Bereich von 0,2 bis 2 Millimeter zwischen Verdampfer (6) und Pulverkavität (1) getestet, wobei sich Durchmesser im Bereich von 1 bis 2 Millimeter als besonders geeignet erwiesen. Im Falle eines für Massenproduktion geeigneten Inhalators ist aus Kostengründen anzuraten, den Verdampfer (6) mit geeignet bemessenem Auslass (9c) direkt an die Pulverkavität (1) anzuschließen.

Nach Austritt aus der Düse (3) wird das Pulver von Luftströmung im Mundstück (2) mitgerissen. Bei Labormessungen mit der Testvorrichtung wird dieser Luftstrom, der beim Inhalator durch Einatmung des Patienten erzeugt wird, durch ein System zur Strömungserzeugung nachgestellt. In beiden Fällen kommt eine Luftströmung im Mundstück (2) durch einen Luftsog am Ende des Mundstück (2) dadurch zustande, dass Luft an einer entgegen gesetzten Stelle in das Mundstück (2) oder in das Gerät durch mindestens eine Einlassöffnung (2b) eintreten kann. Bevorzugt weist der Inhalator bzw. die Testvorrichtung im Bereich von Düse (3) und Mundstück (2) ein oder mehrere mit Einlassöffnungen (2b) verbundene Kanäle auf, die so im Mundstück (2) einmünden, dass die durch die Kanäle strömende Luft den aus der Düse (3) austretenden Treibmittelstrom einhüllt und so die Bestandteile in besonders geeigneter Weise mitnimmt. Insbesondere bilden diese Kanäle einen Bypass zur Düse. Der aerodynamische Durchmesser dieses Bypasses - insbesondere an dessen engster Stelle - bestimmt hierbei den Einatemwiderstand, den ein Patienten beim Inhalieren aus einem analog aufgebauten Inhalator verspürt. Mit der Testvorrichtung wurden Messungen durchgeführt, in denen ein Luftsog mit Flussraten von 30, 60 und 90 Liter pro Minute am Mundstück (2) angelegt und somit ein unterschiedliches Einatemverhalten von Patienten nachgestellt wurde. (30 Liter pro Minute gelten hierbei gemäß der Anweisungen im Europäischen Arzneibuch für die aerodynamische Beurteilung von Dosieraerosolen, 90 Liter pro Minute entsprechen einem Unterdruck von 4 Kilopascal bei einem passiven Pulverinhalator.) Die FPD-Ergebnisse belegten, dass für dieses Zerstäubungskonzept (Ausbringung von trockenem Pulver durch einen länglichen, geradlinigen Düsenkanal mittels in einem Verdampfer getrocknetem Treibmittel) keine nennenswerte Abhängigkeit der Zerstäubung vom Einatemverhalten besteht. Bezogen auf die insgesamt aus dem Zerstäuber ausgebrachte Wirkstoffmenge wurden bei der Flussrate von 30 Litern pro Minute etwas stärkere Ablagerungen im Eingangsbereich des angeschlossenen Messgeräts festgestellt (dieser Eingangsbereich korrespondiert unter Einschränkungen mit dem Mund- und Rachenraum eines Patienten, wenn man diese Messergebnisse auf die Anwendung eines Inhalators überträgt). Bei den Flussraten von 60 und 90 Litern pro Minute zeigte das Zerstäubungsverhalten hinsichtlich der insgesamt ausgebrachten Wirkstoffmenge keinen signifikanten Unterschied. Diese höheren Flussraten sind offensichtlich besser geeignet, auch die größeren Partikel des Aerosols mitzureißen. Insgesamt zeigen die Zerstäubungsdaten für das hier vorgestellte Konzept eine deutlich geringere Flussraten-Abhängigkeit als die meisten kommerziell erhältlichen Pulverinhalatoren.

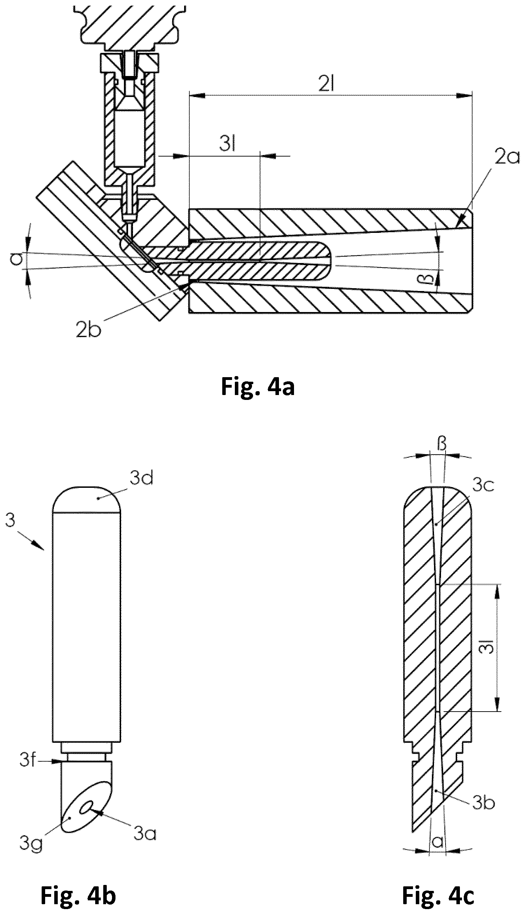

Bevorzugt ist - wie auch in Fig. 4a zu sehen - die Düse (3) bzw. das sie bildende Modul in eine Durchführung (2a) am Mundstück (2) eingeschoben und die Kanäle befinden sich zwischen dem Modul, das die Düse (3) bildet, und einer inneren Wand des Mundstücks (2). Besonders bevorzugt sind Düse und Mundstück im Wesentlichen radialsymmetrisch gestaltet und so zueinander angeordnet, dass ein im Wesentlichen ringförmiger Kanal - allenfalls unterbrochen durch Halterungselemente zwischen Düse (3) und Mundstück (2) - zwischen Düse (3) und Mundstück (2) vorliegt. Optional verjüngt sich die Ringstärke des Kanals zur Einlassöffnung (2b) hin. Dies wird bei einer nicht dargestellten Ausführungsform noch verstärkt, in der die Düse ebenfalls außen eine Konusform aufweist, wobei die Konusneigung der Düse (3) entgegengesetzt zur Konusneigung der Durchführung (2a) am Mundstück (2) ausgebildet ist (d.h. die breiteste Stelle des die Düse (3) bildenden Bauteils sitzt an der schmalsten Stelle der Durchführung (2a)). Bevorzugt hat die Durchführung (2a) am Mundstück (2) eine Konusform, wobei sie sich insbesondere mit einem Winkel von 0° bis 35° und besonders bevorzugt mit einem Winkel von 0° bis 15° wie beispielsweise 5° zu dem Ende des Mundstücks (2) hin öffnet, an dem bei Verwendung bzw. im Betrieb der Sog angelegt wird. Messungen des Feinanteils (FPD) ergaben, dass sich der Feinanteil bei Verwendung der kleineren Konuswinkeln im Mundstück (im Vergleich zur Verwendung der größeren) erhöht, d.h. das die Lungengängigkeit der zerstäubten Partikel besser wird. Bevorzugt ist die Öffnung des Mundrohrs (2) an der Stelle des Auslass der Düse deutlich größer als die Öffnung des Düsen-Auslasses, bevorzugt mindestens 5 mal so groß bezogen auf die Durchmesser. Dies begünstigt die Aerodynamik im Mundrohr.
Bevorzugt ist das Mundstück (2) an besagtem Ende länger als die eingeschobene Düse (3). Bei variierenden Längen (2l) des Mundstücks (2) von insgesamt 15 bis 120 Millimetern wurde (bei festgehaltener kürzerer Länge der Düse (3) von vorzugsweise maximal 15 Millimetern) festgestellt, dass bei den kürzeren Mundstücken zwar geringere Formulierungs-Ablagerungen an der Wand der Durchführung (2a) auftraten, dafür jedoch bei den längeren Mundstücken die Zerstäubungswolke eine bessere Aerodynamik aufwies. Bei den längeren Mundstücken (2) (insbesondere bei denen mit einer Länge (2l) von 120 Millimetern) zeigten Messungen eine Erhöhung des Feinanteils (FPD). Vermutlich kommt es durch die Strömung im Mundrohr zu einem günstigen Abbremsen der durch das Treibgas beschleunigten Partikel, so dass sich bei Anwendung eines solchen Zerstäubers die mögliche Deposition dieser Partikel in der Lunge des Patienten erhöht.
Bei diesen beiden gegenläufigen Effekten - der Bildung von Ablagerungen innen im Mundstück (2) und der Erhöhung des Feinanteils - ergibt sich ein bevorzugter Längenbereich für das Mundstück (2), nämlich 30 - 90 Millimeter, besonders bevorzugt 60 bis 90 Millimeter, oder einen Überstand des Mundstücks (2) gegenüber dem Ende der Düse (3) von 20 bis 70 Millimeter, besonders bevorzugt von 40 Millimeter.

Fig. 4b und Fig. 4c zeigen die Düse (3) im Detail. Sie weist einen zentralen Düsenkanal (3a) auf, der sich zu Beginn in einen Einlasskonus (3b) mit einem Einlasswinkel a und zum Ende hin in einen Auslasskonus (3c) mit Auslasswinkel β öffnet. α und β betragen bevorzugt beide 5°, aber auch andere, von einander Abweichende Winkelgrößen sind möglich. Zwischen diesen beiden Konussen weist der Düsenkanal (3a) einen so genannten zylindrischen Bereich auf, in dem der Querschnitt über eine definierte Länge (3l) konstant ist. Die Anschlussseite (3g) mit der die Düse (3) in der dargestellten Ausführungsform der Testvorrichtung an die Pulverkavität (1) angelegt wird, ist entsprechend der Geometrien des Anschlussstücks (4) gegenüber der durch den Düsenkanal (3a) gebildeten Achse abgewinkelt - hier vorzugsweise um 45°. Bei diesem Winkel trifft zuvor auch das Treibmittel in einem 45° Winkel auf die Pulverkavität (1) aber auch andere insbesondere flachere Winkel sind möglich. Vorteilhaft ist es, hierbei den Abgang des Düsenkanals (3a) von der Pulverkavität (1) spiegelbildlich im gleichen Winkel wie die Zuleitung des Treibmittels auszulegen. Die Düse (3) wird im Ausführungsbeispiel der Testvorrichtung so ins Anschlussstück (4) eingesetzt, dass sie mittels einer in die Sicke (3f) eingesetzten Dichtung derart abgedichtet wird, dass es nicht zu Bypass-Strömungen außen an der Düse (3) in die Pulverkavität (1) kommt. Die Länge des Düsenkanals und die Länge (3l) des zentralen zylindrischen Teils des Düsenkanals (3a) sind wichtige Funktionsparameter der Düse (3). Ebenso wie die Länge (2l) des Mundstücks (2) wurde die Länge des Düsenkanals (3a) im Rahmen von Messungen mit der Testvorrichtung von 3 Millimetern bis 15 Millimetern variiert. Auch hier ergab es sich bei Messungen des Feinanteils (FPD), dass sich der Feinanteil bei Verwendung der größeren Längen (insbesondere bei 15 Millimetern) erhöht, d.h. das die Lungengängigkeit der zerstäubten Partikel bei der Verwendung längerer Düsen (3) besser wird. Dies erklärt sich durch die längere Einwirkzeit von Scherkräften auf den Gasstrom bzw. das Aerosol in den längeren Düsenkanälen (3a). Die Einwirkzeit ist insgesamt wegen der hohen Geschwindigkeit des Gasstroms sehr kurz. Fluss-Simulationen zeigten, dass die Geschwindigkeit des Gasstroms in der Düse (3) Werte von bis zu 1 Mach annehmen kann. Die Geschwindigkeit in der Düse ist abhängig vom aerodynamischen Querschnitt der Düse (3). Bei kleinerem Querschnitt der Düsenkanals (3a) erhöht sich der Strömungswiderstand der Düse, die Deagglomeration der Formulierungspartikel nimmt zu. Dies wurde anhand der Ergebnisse von Messungen mit unterschiedlichen Querschnitten von Düsenkanälen (3a) belegt. Getestet wurden Querschnitte im Bereich von 0,2 bis 0,8 Quadratmillimetern (z.B. ein kreisförmiger Durchmesser mit 1 Millimeter Durchmesser). Ausgewertet wurde für diese Messungen insbesondere das Verhältnis von Feinanteil (FPD) zu insgesamt aus dem Zerstäuber ausgebrachter Wirkstoffmenge. Dieses Verhältnis erhöhte sich bei kleiner werdendem Querschnitt. Exemplarisch wurde eine solche Messung auch mit einem Düsenkanal (3a) mit ovalem statt rundem Querschnitt durchgeführt. Bezogen auf die Querschnittsfläche waren die Ergebnisse für das Verhältnis von FPD zu ausgebrachter Wirkstoffmenge für den runden und den ovalen Querschnitt des Düsenkanals (3a) annähernd gleich.
Weitere Messungen mit unterschiedlichen Düsenkanälen (3a) zeigen neben der besseren Deagglomeration von Partikeln bei kleinen Querschnitten der Düsenkanäle (3a) jedoch einen weiteren Effekt auf: Die insgesamt aus dem Zerstäuber ausgebrachte Formulierungsmenge zeigt eine eigene Abhängigkeit vom Querschnitt des Düsenkanals (3a). Bei einer Querschnittsfläche von 0,2 Quadratmillimeter zeigt sie den im Vergleich niedrigsten Wert (in der konkreten Messreihe 62% bezogen auf die in der Pulverkavität (1) eingefüllte Formulierungsmenge), bereits bei 0,4 Quadratmillimeter wird ein deutlich höherer Wert (73%) erreicht, der zu wiederum höheren Querschnitten zunächst noch leicht zunimmt (76% bei 0,5 Quadratmillimetern) und anschließend tendenziell wieder leicht abnimmt (73% bei 0,8 Quadratmillimetern). Bei der Verwendung von Düsen (3) mit sehr engen Düsenkanälen (3a) wird weniger Pulver aus dem System ausgebracht, und stattdessen bleibt mehr Pulver in der Pulverkavität (1) zurück. Bevorzugt werden hier also Querschnitte im Bereich von 0,4 bis 0,7 Quadratmillimetern für den Düsenkanal (3a). Exemplarische Messungen deuten darauf hin, dass sich die insgesamt aus dem Zerstäuber ausgebrachte Formulierungsmenge durch die Verwendung von ovalen Querschnitten erhöht.

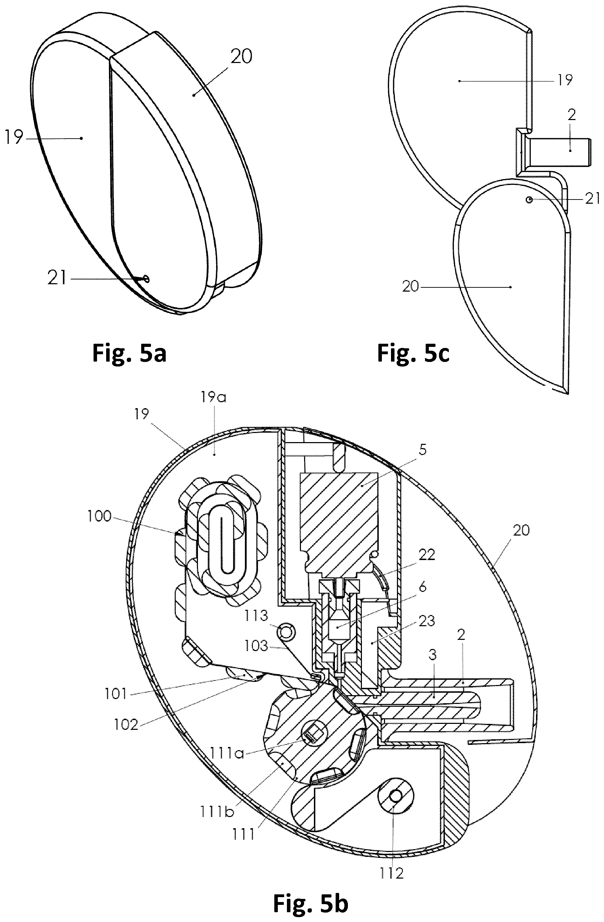

Fig. 5 zeigt einen erfindungsgemäßen Zerstäuber, der als Inhalator von einem Patienten angewendet werden kann und als kompaktes Handgerät ausgelegt ist. Die Funktionsweise dieses Inhalators ist die gleiche wie die anhand der Testvorrichtung aus Fig. 2 beschriebene. Insbesondere sind alle vorangegangenen Schilderungen, die das Mundstück (2). die Düse (3), die Kartusche (5) und den Verdampfer (6) betreffen, ebenfalls auf den in Fig. 5 gezeigten Inhalator anzuwenden. Wie Fig. 5 zu entnehmen ist sind diese Komponenten ganz analog wie im Ausführungsbeispiel gemäß Fig. 2 angeordnet.
Fig. 5a zeigt das Äußere des hier ausgeführten Inhalators mit einer geschlossenen Mundstückabdeckung, Fig. 5c das Äußere mit einer geöffneten Mundstückabdeckung. Der Inhalator weist als besagte Mundstückabdeckung eine Abdeckung (20) auf, die über eine Drehachse (21) mit einem Gehäuse (19) verbunden ist. Wenn der Inhalator nicht benutzt wird, kann die Abdeckung (20) geschlossen werden und verdeckt das Mundstück (2) des Inhalators. In diesem Transportzustand mit geschlossener Abdeckung (20) sind nur Gehäuse (19) und Abdeckung (20) von außen zugänglich, und alle für die Zerstäubung funktionsrelevanten Bauteile sind gegen Verschmutzungen geschützt. Die Anbindung der Abdeckung (19) über eine hier pivotal ausgeführte Drehachse (21) ermöglicht ein einfaches Öffnen des Inhalators und stellt sicher, dass die Abdeckung (20) am Inhalator verbleibt und nicht verloren gehen kann.
Fig. 5b zeigt einen schematischen Schnitt des Inhalators, der in diesem Ausführungsbeispiel als pmDPI ausgelegt ist, d.h. als Mehrdosis-Gerät mit einzelnen vorabgemessenen und einzeln bevorrateten Dosiseinheiten einer pulverförmigen Formulierung. Die einzelnen Dosiseinheiten sind hier bevorzugt auf streifenförmigen Trägern angeordnet, insbesondere einem sogenannten Blisterband (100), das hintereinander gereihte Blisterkavitäten (101) aufweist, die zwischen einer Trägerbahn (102) und einer Deckfolie (103) gebildet werden. Insbesondere weist die bevorzugt aus einem Kunststoff und/oder Aluminium gefertigte Trägerbahn hierzu Vertiefungen auf, die z.B. in einem Tiefziehprozess gefertigt wurden. Die Blisterkavitäten (101) sind mit der pulverförmigen Formulierung gefüllt und können schrittweise mittels eines Rads (111) an eine Position gebracht werden, in der sie jeweils die Funktion der Pulverkavität (1) in einer funktionalen Anordnung analog zu der Testvorrichtung in den vorangegangenen Figuren übernehmen. Das Rad (111) weist hierzu entlang seines äußeren Umfangs äquidistant verteilt napf- oder taschenförmige Aufnahmen (111 b) auf, welche die Blisterkavitäten (101) auf der Seite ihrer Trägerbahn (102) aufnehmen können und jeweils in die Pulverentnahmeposition mit Anschluss an die Treibmittelzuleitung aus dem Verdampfer (6) und an die Düse (3) drehen. An der Pulverentnahmeposition weist das Gerät eine Abdichtung auf, die verhindert, das Treibgas an der Blisterkavität (101) vorbei ausweichen kann und nur durch die Düse wieder austritt. Bevorzugt wird vor Erreichen der Pulverentnahmeposition die Deckfolie (103) von der Trägerbahn abgezogen und so geöffnet. Die Deckfolie wird vorzugsweise auf einer Spule (113) aufgewickelt. Bezogen auf den Vortrieb des Blisterbands (100) wird die Deckfolie (103) erst so spät von der Trägerbahn (102) separiert, dass immer nur die Blisterkavität (101), die zur gerade Pulverentnahmeposition gebracht wird, geöffnet ist und keine weiteren Blisterkavitäten (101), die noch Pulver enthalten, geöffnet werden. Die Trägerbahn (102) mit den entleerten Vertiefungen der Blisterkavitäten (101) wird auf einer weiteren Spule (112) aufgewickelt. Hierzu wird sie bevorzugt zwischen Pulverentnahmeposition und Spule (112) durch eine - nicht in der Abbildung gezeigte - Vorrichtung geführt, in der die Trägerbahn (102) geglättet und/oder die in ihr enthaltenen Vertiefungen platt gedrückt werden. Eine solche Vorrichtung ist z.B. der Schrift WO2007096111A2 (Seite 5 und den Ausführungen zu den dortigen Figuren 2 und 4) zu entnehmen.

An der Pulverentnahmeposition wird die geöffnete Blisterkavität (101) dicht an das Anschlussstück, das die Treibmittelzuführung und den Einlass des Düsenkanals (3a) aufweist, herangeführt oder angedrückt. Beispielsweise ist das Rad (111), das Blisterband (100) an dieser Stelle so orientiert, dass die Blisterkavität (101) gegen das Anschlussstück gedrückt oder gezwängt wird. Die Aufnahmen (111b) am Rad (111) sind dabei derart gestaltet, dass die Oberseite der Blisterkavität (101) die gleiche Wölbung wie die zugehörige Unterseite des Anschlussstücks aufweistbevorzugt liegt die Blisterkavität (101) komplett flach auf dem Rad (111) auf und ist nicht gewölbt. Bevorzugt beinhaltet das Anschlussstück Materialien, die insbesondere den oberen äußeren Rand der geöffneten Blisterkavität (101) gegen das Anschlussstück abdichten, wie eine Teflonbeschichtung oder einen in die Kontaktoberfläche eingelassenen Dichtungsring.

Zusätzlich ist die Drehachse (111a) des Rads (111) bevorzugt derart durch eine Feder oder ähnliches mit Druck beaufschlagt, so dass das Rad (111) in Richtung des Anschlussstücks gedrückt und auf diese Weise die Dichtigkeit gewährleistet wird.

Alternativ zum Rad (111) kann eine vorzugsweise federvorgespannte Führungsschiene verwendet werden. Über eine Zugkraft an der Spule (112) wird dann zunächst die Blisterkavität (101) in ihre Position am Anschlussstück gebracht und anschließend durch diese Führungsschiene angedrückt. Je nach Gestaltung des Blistersbandes (100) kann die Führungsschiene dabei eine glatte Oberfläche haben (dann eignet sie sich auch zur Führung des Blisterbandes beim Weitertransport im Gerät) oder eine bewegliche Druckplatte mit Aufnahme sein, wobei diese Platte während des Transport des Blisterbandes (100) dieses nicht berührt.

In einer Ausführungsform des erfindungsgemäßen Zerstäubers entspricht der Mechanismus zum Abziehen der Deckfolie (103) vorzugsweise dem entsprechenden Mechanismus, der in der Schrift DE4106379A1 offenbart wird.

Eine Weiterentwicklung dieses Transportmechanismus findet sich in der EP1436216B1.

Bevorzugt wird der Vortrieb des Blisterbandes (100) durch eine Bewegung der Abdeckung (20), vorzugsweise durch die Öffnungsbewegung, bewirkt. Hierzu ist die Drehachse (21) vorzugsweise an die Spule (112) gekoppelt, so dass beim Öffnen des Geräts das Blisterband (100) in Vortriebsrichtung gezogen wird und sich das Rad (111) dabei mit dreht. Bei dieser Kopplung wird dann eine Rücklaufsperre beispielsweise in Form einer Rutschkupplung in der Art vorgesehen, dass die Drehbewegung der Achse (21) nur in einer Richtung (vorzugsweise der Öffnungsrichtung) auf die Spule (112) übertragen wird. Zusätzlich wird optional die Drehachse (21) und /oder Spule (112) über ein - in Fig. 5 nicht dargestelltes - Getriebe mit der Achse (111 a) des Rads (111) und/oder der Spule (113) zum Aufwickeln der Deckfolie (103) gekoppelt. Hierbei besteht auch die Möglichkeit, dass die Drehachse (21) direkt auf die Achse (111 a) des Rads (111) wirkt und dies über ein Getriebe seinerseits mit den beiden Spulen (112, 113) verbunden ist. Bezüglich des möglichen Aufbaus eines solchen Getriebes und der Rücklaufsperre sei hier auf die Schrift WO2007134792A1 (Seite 4 Zeile 30-34, Seite 6 Zeile 30 bis Seite 7 Zeile 13, Seite 8 Zeile 7 bis 29, Seite 9 Zeile 21 bis 29 und Seite 10 Zeile 25 bis Seite 14 Zeile 13) verwiesen.

Die Rücklaufsperre bezüglich der Übertragung der Drehbewegung der Drehachse (21) und der Achse (111 a) des Rads (111) und/oder der Spulen (112, 113) kann des Weiteren entsprechend der Rücklaufsperre gestaltet werden, die in der Schrift WO07068896 offenbart wird.

Alternativ zum Abziehen der Deckfolie (103) und der Verwendung einer zugehörige Spule (113) können die Blisterkavitäten (01) auch vor Erreichen der Pulverentnahmeposition an einer anders gearteten Öffnungsvorrichtung vorbei geführt werden, an der die Deckfolie (103) an der Stelle der Blisterkavität (101) beispielsweise angestochen oder aufgeschnitten oder anderweitig geöffnet wird.

Bevorzugt ist der Inhalator so gestaltet, dass sich Blisterband (100), Rad (111) und die Spulen (112, 113) und gegebenenfalls zwischen diesen wirkende Getriebe-Elemente in einem austauschbaren Gehäuseteil (19a) befinden. Auf diese Weise wird die Größe des Inhalators nicht durch die Länge des Blisterbandes (100), d.h. nicht durch die maximal mögliche Anzahl von Dosierungen bestimmt. Zur Ausbringung des Pulvers aus der in die Pulverentnahmeposition gebrachten Blisterkavität (101) wird Treibmittel aus der Kartusche (5) abgegeben. Dies kann entweder dadurch geschehen, dass der Anwender bzw. Patient direkt auf die Kartusche (5) - an ihrem Boden, der sich auf der dem Ventilstamm (7) entgegengesetzten Seite befindet - in Richtung ihres in dem Fall bevorzugt gefedert gelagertenVentilstammes (7) drückt oder dadurch, dass der Patient durch Einatmen am Mundstück (2) eine entsprechende Bewegung der Kartusche (5) auslöst. Eine solche so genannte Atemzugstriggerung ist in dem in Fig. 5a skizzierten Gerät vorgesehen.

Fig. 5 zeigt eine verkippbare Fahne (22), welche die Kartusche (5) in einer Ruheposition leicht von der Ventilstammaufnahme (8a) beabstandet, so dass das Ventil der Kartusche (5) geschlossen ist. Atmet der Patient nun am Mundstück (2) ein, so erzeugt er insbesondere an der Einlassöffnung (2b) der Mundstücks einen Sog, der sich ebenfalls auf den daran angeschlossenen Hohlraum erstreckt, der im Ausführungsbeispiel einen Bypass (23) darstellt, in dem Luft neben dem Verdampfer vorbei strömen kann. Die mit der Umgebungsluft verbundene Einlassöffnung dieses Bypasses ist in Ruheposition durch einen Teil der Fahne (22) verschlossen. Durch den Sog beim Einatemnvorgang kippt die Fahne (22) derart, dass an ihrem einen Ende die Einlassöffnung am Bypass (23) freigegeben wird. Dadurch wird eine Blockierung der Kartusche (5) gelöst und der Bewegungsweg für die Kartusche (5) am anderen Ende der Fahne (22) wird nach unten bzw. in Richtung der Ventilstammaufnahme (8a) freigegeben. Bevorzugt ist die Kartusche (5) derart federvorgespannt gelagert, dass sie sich selbst bei Auslenkung der Fahne (22) beim Einatemvorgang in Richtung der Ventilstammaufnahme (8a) bewegt. Lässt der durch das Einatmen am Mundstück (2) erzeugte Sog im Bypass (23) nach, stellt sich die Fahne (22) in ihre ursprüngliche Position zurück und die Kartusche (5) wird wieder von der Ventilstammaufnahme (8a) beabstandet. Je nach Stärke der Atemzugstriggerung kann die Fahne (22) hierzu auch mit einem Rückstellmechanismus verbunden sein. Nach der Atemzugsauslösung der Bewegung der Kartusche (5) wird die Kartusche vorzugsweise beim Schließen oder Öffnen der Abdeckung (20) - vorzugsweise über eine Kulissenführung - wieder in die vorgespannte Ausgangssituation gebracht.

Eine solche Kombination aus Vorspannung einer Treibmittel-Kartusche und einer Atemzugstriggerung wird in der US5031610 offenbart. In der US5031610 wird die Vorspannung der Kartusche und die Vorbereitung der Atemzugstriggerung durch Absetzen und erneutes Aufstecken einer Kappe auf dem Mundstück verursacht. In einer hier bevorzugten Ausführungsform würde der Mechanismus aus der US5031610 statt dessen an die Schwenkbewegung der Abdeckung (20) oder an einen zusätzlichen, nicht gezeigten Hebel gekoppelt werden.

Alternativ zu einer rein mechanischen Atemzugstriggerung können auch elektromechanische Steuerungen verwendet werden. In solchen (nicht gezeigten) Ausführungsformen weist der Zerstäuber vorzugsweise eine Batterie auf, welche die für solche Steuerungen benötigten elektrischen Spannungen zur Verfügung stellt. Bei einer solchen elektrischen oder elektromechanischen Atemzugstriggerung weist der Zerstäuber innen am Mundstück (2) einen elektrischen Flusssensor auf, der in Abhängigkeit von der detektierten Strömung ein mit der Flussrate variierendes elektrisches Signal abgibt. Dieses Signal wird dann verwendet um einen elektromechanischen Vorgang zu starten, durch den z.B. die Kartusche (5) in Richtung der Ventilstammaufnahme (8a) bewegt wird, das Ventil der Kartusche (5) geöffnet und somit Treibmittel in den Verdampfer (6) bzw. die Kanäle des Zerstäubers freigesetzt wird. Damit diese Ventilbetätigung erst bei einer vordefinierten Luftströmung, d.h. einem bestimmten Sog am Mundstück (2), stattfindet wird das Sensor-Signal zuvor durch eine Kontrollvorrichtung beispielsweise eine analoge Vergleichsschaltung oder eine digitale Elektronik geleitet. Das Sensor-Signal löst gewissermaßen bei Erreichen eines bestimmten Sogs am Mundstück (2) einen elektrischen Schalter aus. Bei Betätigung dieses elektrischen Schalters wird ein elektromechanischer Vorgang gestartet, beispielsweise ein Schrittmotor in Gang gesetzt, der die Kartusche verschiebt. Solche elektromechanischen Atemzugstriggerungen von Zerstäubern mit Treibmittel-Kartuschen sind in der Schrift WO9207599A1 offenbart.

Eine wiederum andere Möglichkeit eine Atemzugstriggerung im Zerstäuber einzubringen besteht darin, dass zusätzlich zu dem zur Kartusche (5) gehörenden Ventil ein zweites Ventil vorgesehen wird. Das zweite Ventil befindet sich bei einer solchen Ausführungsform strömungstechnisch gesehen vor dem Einlass des Verdampfers (6). Der Zerstäuber wird für die Inhalation derart vorbereitet, dass das 1. Ventil betätigt wird - z.B. durch eine Verschiebung der Kartusche (5), wobei die Verschiebung an das Öffnen der Abdeckung (20) gekoppelt ist - und das abgegebene Treibmittel fließt in eine Vorkammer vor dem zweiten Ventil. Bei Auslösung der Atemzugstriggerung wird dann lediglich das 2. Ventil geöffnet, was eine geringere Krafteinleitung benötigt als die für die Auslösung des ersten Ventils benötigte Verschiebung der Kartusche (5). Eine solche Ausführungsform mit einem zweiten Ventil zusätzlich zu dem Dosierventil einer Kartusche (5) kann eine Ventilanordnung und deren Kopplung mit einer Atemzugssteuerung aufweisen, wie sie in der Schrift GB2233236A offenbart wird, die auf die Atemzugstriggerung eines MDI gerichtet ist.

Das zweite Ventil wird direkt durch den Sog des Patienten geöffnet, z.B. durch das Ansaugen eines Tellers oder Stempels, der mit der Öffnung des zweiten Ventils verbunden ist oder durch das indirekten Ansaugen einer Komponente, die zu einem ansonsten durch magnetische Kräfte verschlossenen zweiten Ventil gehört. Ein solcher Teller oder Stempel wird in der hier betrachteten Ausführungsform des Zerstäubers bevorzugt im Bypass (23) vorgesehen.

Eine weitere, alternativ ebenfalls so einer Ausführungsform des erfindungsgemäßen Zerstäubers mit Atemzugstriggerung verwendbare Anordnung eines zweiten Ventils zusätzlich zu dem Dosierventil der Kartusche (5) wird in der Schrift EP0476991A1 offenbart.

Bei dieser Ausführungsform ist dann der z.B. in den Bypass (23) hineinragende Flusssensor - gegebenenfalls einstückig - mit einem federvorgespannten Kolben verbunden, der bei Auslenkung gegen die Federvorspannung das zweite Ventil öffnet, so dass Treibmittel aus der Vorratskammer in den Verdampfer fließen kann.

Bevorzugt werden die hier beschriebenen Zerstäuber mit einer medizinischen Formulierungen betrieben, die ein Bestandteil aus der Offenbarung der europäisehen Patentanmeldung mit der Anmeldenummer 12151105.9 auf Seite 26 Zeile 12 bis Seite 63 Zeile 2 aufweist oder einer der dort genannten Formulierungen entspricht.

### Bezugszeichenliste

- 1: Pulverkavität
- 2: Mundstück
- 2a: Durchführung (am Mundstück)
- 2b: Einlassöffnung (am Mundstück)
- 2l: Länge (des Mundstücks)
- 3: Düse
- 3a: Düsenkanal
- 3b: Einlasskonus (der Düse)
- 3c: Auslasskonus (der Düse)
- 3d: Endfläche (der Düse)
- 3g: Anschlussseite (der Düse)
- 3f: Sicke
- 3l: Länge (des zylindrischen Teils des Düsenkanals)
- 4: Anschlussstück
- 5: Kartusche
- 6: Verdampfer
- 7: Ventilstamm
- 8: Deckel (am Verdampfer)
- 8a: Ventilstammaufnahme (im Deckel)
- 8b: Flansch (am Deckel)
- 8c: Sicke (im Flansch)
- 8d: nnenkonus (am Deckel)
- 9: Körper (des Verdampfers)
- 9a: Hohlraum (des Verdampfers)
- 9b: Trichter
- 9c: Auslass (des Verdampfers)
- 9d: Stamm (am Verdampfer)
- 10: Dichtung
- 11: Kugel
- 12: Draht
- 19: Gehäuse
- 19a: austauschbares Gehäuseteil
- 20: Abdeckung (für Mundstück)
- 21: Drehachse (für Abdeckung)
- 22: Fahne
- 23: Bypass
- 100: Blisterband
- 101: Blisterkavität
- 102: Trägerbahn
- 103: Deckfolie
- 111: Rad
- 111a: Drechachse (an Rad)
- 111b: Aufnahme (an Rad)
- 112: Spule (für Trägerbahn)
- 113: Spule (für Deckfolie)

- α: Einlasswinkel (an Düse)
- β: Auslasswinkel (an Düse)

## Patentansprüche

1. Gerät zur Zerstäubung von pulverförmigen medizinischen Formulierungen, bei dem die Zerstäubung durch ein Treibmittel aus einer Kartusche (5) unterstützt wird, in der das Treibmittel in verflüssigter Form vorliegt,
wobei das Treibmittel einer Kavität zugeführt wird, in der sich eine abgemessene Menge der Formulierung befindet, wobei das Gerät einen Verdampfer (6) aufweist, durch den das Treibmittel vor seiner Zuführung in die Kavität geleitet wird, und wobei der Verdampfer (6) einen Hohlraum (9a), einen Einlass für Treibmittel und einen Auslass (9c) aufweist,
wobei von der Kavität aus ein mit Formulierung beladener Treibmittelstrom in eine Düse (3) geleitet wird und die Düse (3) einen im Wesentlichen geradlinigen Düsenkanal (3a) aufweist,
**dadurch gekennzeichnet, dass**
die Zuleitung von Treibmittel in die Kavität und die Achse durch den Düsenkanal (3a) in einem gleich großen Winkel in der Kavität eintreffen, wobei der Winkel 30°, zwischen 30° und 45° oder 45° relativ zum Boden der Kavität ist.

2. Gerät nach Anspruch 1 **dadurch gekennzeichnet, dass** sich im Hohlraum (9a) des Verdampfers (6) ein oder mehrere Wärmetausch-Elemente befinden, wobei das mindestens eine Wärmetausch-Element aus Metall ist und die Oberfläche des mindestens einen Wärmetausch-Elements die innere Oberfläche des Verdampfers (6) vergrößert.

3. Gerät nach Anspruch 2 **dadurch gekennzeichnet, dass** sich mehrere Wärmetausch-Elemente im Verdampfer (6) befinden und Metallkugeln und/oder Metalldrähte die Wärmetausch-Elemente bilden.

4. Gerät nach einem der vorangegangenen Ansprüche **dadurch gekennzeichnet, dass** alle Bauteile des Verdampfers (6) aus Metall sind.

5. Gerät nach einem der vorangegangenen Ansprüche **dadurch gekennzeichnet, dass** im Innern des Verdampfers (6) die Bauteile des Verdampfers (6) derart gestaltet sind, dass sie bei Durchströmung des Verdampfers (6) mit Treibmittel einen möglichst geringen Strömungswiderstand darstellen, indem der Verdampfer (6) rotationssymmetrisch ist und Übergänge im Einlass- und Auslassbereich des Hohlraums (9a) des Verdampfers (6) konusförmig sind.

6. Gerät nach einem der vorangegangenen Ansprüche **dadurch gekennzeichnet**, das es derart gestaltet ist, dass ein mit Formulierung beladener Treibmittelstrom von der Kavität aus in eine Düse (3) geleitet wird, deren Öffnung in ein in Strömungsrichtung im Vergleich zur Düse (3) verlängertes Mundstück (2) einmündet und mindestens eine Einlassöffnung (2b) am Mundstück (2) eine Bypass-Luftströmung nahe dem Auslass der Düse (3) ermöglicht.

7. Gerät nach einem der vorangegangenen Ansprüche **dadurch gekennzeichnet, dass** der Düsenkanal (3a) einen Einlasskonus (3b) und/oder einen Auslasskonus (3c) aufweist und/oder die Wand des Düsenkanals (3a) zumindestens in einem zentralen Bereich zylinderförmig ist.

8. Gerät nach einem der vorangegangenen Ansprüche **dadurch gekennzeichnet, dass** die Länge des Düsenkanals (3a) mindestens 8 Millimeter, bevorzugt mindestens 15 Millimeter beträgt.

9. Gerät nach einem der vorangegangenen Ansprüche **dadurch gekennzeichnet, dass** der Düsenkanal (3a) an seiner engsten Stelle einen Querschnitt von 0,2 bis 0,8 Quadratmillimetern, bevorzugt von 0,4 bis 0,7 Quadratmillimetern hat.

10. Gerät nach einem der vorangegangenen Ansprüche **dadurch gekennzeichnet, dass** der Düsenkanal (3a) einen kreisförmigen oder ovalen Querschnitt aufweist.

11. Gerät nach einem der vorangegangenen Ansprüche **dadurch gekennzeichnet, dass** der Durchmesser der Öffnung eines Mundstücks (2), in das die Düse (3) einmündet, an der Stelle des Auslass der Düse (3) mindestens fünfmal so groß ist wie der Durchmesser der Öffnung des Auslasses der Düse (3).

12. Gerät nach einem der vorangegangenen Ansprüche **dadurch gekennzeichnet**, das es derart gestaltet ist, dass ein mit Formulierung beladener Treibmittelstrom von der Kavität aus in eine Düse (3) geleitet wird, deren Öffnung in ein Mundstück (2) einmündet und dass das Mundstück (2) um eine Länge von 15 bis 70 Millimeter, bevorzugt von 40 bis 70 Millimeter über das in das Mundstück (2) eingeschobene Ende der Düse (3) hinausragt.

13. Gerät nach einem der vorangegangenen Ansprüche **dadurch gekennzeichnet, dass** das Gerät ein Inhalator zur Ausbringung mehrerer einzeln abgemessener Mengen der vorzugsweise pulverförmigen Formulierung ist, wobei die einzelnen Mengen der Formulierung sich vor ihrer Ausbringung in den Kavitäten eines Blisterbands (100), besonders bevorzugt in den Kavitäten eines Blisterbands (100) mit abtrennbarer Deckfolie (103), befinden.

14. Gerät nach einem der vorangegangenen Ansprüche **dadurch gekennzeichnet, dass** das Gerät ein Inhalator mit einer Atemzugstriggerung ist, wobei die Atemzugstriggerung die Zuführung von Treibmittel in den Verdampfer (6) auslöst.

## Claims

1. Device for nebulising powdered medicinal formulations, the nebulisation being assisted by means of a propellant from a cartridge (5) in which the propellant is contained in liquid form, the propellant being fed into a cavity in which there is a measured amount of the formulation, the device comprising a vaporiser (6) through which the propellant is conveyed before being fed into the cavity, and the vaporiser (6) comprising a hollow space (9a), an inlet for the propellant and an outlet (9c), a formulation-laden propellant stream being conveyed from the cavity into a nozzle (3), and the nozzle (3) comprising a substantially straight nozzle channel (3a), **characterised in that** the propellant conveyed into the cavity and the axis through the nozzle channel (3a) enter the cavity at the same angle, the angle being 30°, between 30° and 45°, or 45° relative to the base of the cavity.

2. Device according to claim 1, **characterised in that** one or more heat exchange elements are located in the hollow space (9a) of the vaporiser (6), the at least one heat exchange element being made of metal and the surface area of the at least one heat exchange element increasing the inner surface area of the vaporiser (6).

3. Device according to claim 2, **characterised in that** a plurality of heat exchange elements are located in the vaporiser (6) and the heat exchange elements are formed by metal balls and/or metal wires.

4. Device according to any of the preceding claims, **characterised in that** all the components of the vaporiser (6) are made of metal.

5. Device according to any of the preceding claims, **characterised in that** the components of the vaporiser (6) are laid out in the interior of the vaporiser (6) in such a way that there is as little flow resistance as possible when propellant flows through the vaporiser (6) since the vaporiser (6) is rotationally symmetrical and transitions in the inlet and outlet region of the hollow space (9a) of the vaporiser (6) are conical.

6. Device according to any of the preceding claims, **characterised in that** it is designed in such a way that a formulation-laden propellant stream is conveyed from the cavity into a nozzle (3), the opening of which leads into a mouthpiece (2) that is extended relative to the nozzle (3) in the flow direction, and at least one inlet opening (2b) on the mouthpiece (2) allows for a bypass airflow close to the outlet of the nozzle (3).

7. Device according to any of the preceding claims, **characterised in that** the nozzle channel (3a) comprises an inlet cone (3b) and/or an outlet cone (3c) and/or the wall of the nozzle channel (3a) is cylindrical in at least a central region.

8. Device according to any of the preceding claims, **characterised in that** the length of the nozzle channel (3a) is at least 8 mm, preferably at least 15 mm.

9. Device according to any of the preceding claims, **characterised in that** the nozzle channel (3a) has a cross section at its narrowest point of from 0.2 to 0.8 mm², preferably of from 0.4 to 0.7 mm².

10. Device according to any of the preceding claims, **characterised in that** the nozzle channel (3a) has a circular or oval cross section.

11. Device according to any of the preceding claims, **characterised in that**, at the site of the outlet of the nozzle (3), the diameter of the opening of a mouthpiece (2) into which the nozzle (3) leads is at least five times larger than the diameter of the opening of the outlet of the nozzle (3).

12. Device according to any of the preceding claims, **characterised in that** it is designed in such a way that a formulation-laden propellant stream is conveyed from the cavity into a nozzle (3), the opening of which leads into a mouthpiece (2), and **in that** the mouthpiece (2) protrudes beyond the end of the nozzle (3) inserted into the mouthpiece (2) by a length of from 15 to 70 mm, preferably of from 40 to 70 mm.

13. Device according to any of the preceding claims, **characterised in that** the device is an inhaler for dispensing a plurality of individually measured amounts of the preferably powdered formulation, the individual amounts of the formulation being located in the cavities of a blister tape (100), particularly preferably in the cavities of a blister tape (100) having a removable cover film (103), before being dispensed.

14. Device according to any of the preceding claims, **characterised in that** the device is an inhaler having a breath trigger, the breath trigger initiating the supply of propellant into the vaporiser (6).

## Revendications

1. Appareil pour la pulvérisation de formulations médicinales pulvérulentes, où la pulvérisation est assistée par un agent propulseur issu d'une cartouche (5) dans laquelle l'agent propulseur se présente sous une forme liquéfiée,
dans lequel l'agent propulseur est acheminé à une cavité dans laquelle se trouve une quantité mesurée de formulation, dans lequel l'appareil présente un évaporateur (6) par lequel l'agent propulseur est conduit avant son acheminement dans la cavité, et dans lequel l'évaporateur (6) présente un espace creux (9a), une entrée pour l'agent propulseur et une sortie (9c),
dans lequel un flux d'agent propulseur chargé avec la formulation est conduit de la cavité dans une buse (3) et la buse (3) présente un canal de buse (3a) sensiblement rectiligne,
**caractérisé en ce que**
la conduite de l'agent propulseur dans la cavité et l'axe au travers du canal de buse (3a) arrivent dans un angle de même grandeur dans la cavité, dans lequel l'angle est de 30°, entre 30° et 45° ou 45° par rapport au plancher de la cavité.

2. Appareil selon la revendication 1, **caractérisé en ce que** dans l'espace creux (9a) de l'évaporateur (6) se trouvent un ou plusieurs éléments échangeurs de chaleur, dans lequel l'au moins un élément échangeur de chaleur est en métal et la surface de l'au moins un élément échangeur de chaleur agrandit la surface intérieure de l'évaporateur (6).

3. Appareil selon la revendication 2, **caractérisé en ce que** plusieurs éléments échangeurs de chaleur se trouvent dans l'évaporateur (6) et des billes métalliques et/ou des fils métalliques forment les éléments de l'échangeur de chaleur.

4. Appareil selon l'une des revendications précédentes,
**caractérisé en ce que** tous les composants de l'évaporateur de chaleur (6) sont en métal.

5. Appareil selon l'une des revendications précédentes,
**caractérisé en ce que**, à l'intérieur de l'évaporateur (6), les composants de l'évaporateur (6) sont conçus de telle sorte qu'ils constituent lors de la traversée de l'évaporateur (6) avec l'agent propulseur, une résistance à l'écoulement aussi faible que possible, l'évaporateur (6) étant symétrique en rotation et des passages dans la région d'entrée et de sortie de l'espace creux (9a) de l'évaporateur (6) étant en forme de cône.

6. Appareil selon l'une des revendications précédentes,
**caractérisé en ce qu'**il est conçu de telle sorte qu'un flux d'agent propulseur chargé avec la formulation est conduit de la cavité dans une buse (3) dont l'ouverture débouche dans un embout buccal (2) prolongé dans la direction d'écoulement par rapport à la buse (3) et au moins une ouverture d'entrée (2b) sur l'embout buccal (2) permet un écoulement d'air de dérivation à proximité de la sortie de la buse (3).

7. Appareil selon l'une des revendications précédentes,
**caractérisé en ce que** le canal de buse (3a) présente un cône d'entrée (3b) et/ou un cône de sortie (3c) et/ou la paroi du canal de buse (3a) est au moins de forme cylindrique dans une zone centrale.

8. Appareil selon l'une des revendications précédentes,
**caractérisé en ce que** la longueur du canal de buse (3a) est d'au moins 8 millimètres, de préférence d'au moins 15 millimètres.

9. Appareil selon l'une des revendications précédentes,
**caractérisé en ce que** le canal de buse (3a) a dans son endroit le plus étroit, une section transversale de 0,2 à 0,8 millimètre carré, de préférence de 0,4 à 0,7 millimètre carré.

10. Appareil selon l'une des revendications précédentes,
**caractérisé en ce que** le canal de buse (3a) présente une section transversale circulaire ou ovale.

11. Appareil selon l'une des revendications précédentes,
**caractérisé en ce que** le diamètre de l'ouverture d'un embout buccal (2) dans lequel débouche la buse (3), est au moins cinq fois plus grand à l'endroit de la sortie de la buse (3), que le diamètre de l'ouverture de la sortie de la buse (3).

12. Appareil selon l'une des revendications précédentes,
**caractérisé en ce qu'**il est conçu de telle sorte qu'un flux d'agent propulseur chargé avec la formulation est conduit de la cavité dans une buse (3) dont l'ouverture débouche dans un embout buccal (2) et **en ce que** l'embout buccal (2) dépasse d'une longueur de 15 à 70 millimètres, de préférence de 40 à 70 millimètres au-dessus de l'extrémité de la buse (3) enfoncée dans l'embout buccal (2).

13. Appareil selon l'une des revendications précédentes,
**caractérisé en ce que** l'appareil est un inhalateur pour diffuser plusieurs quantités mesurées individuellement de la formulation de préférence sous forme pulvérulente, dans lequel les quantités individuelles de la formulation se trouvent avant leur diffusion dans les cavités d'une bande thermoformée (100), de manière particulièrement préférée dans les cavités d'une bande thermoformée (100) comportant un film de recouvrement séparable (103).

14. Appareil selon l'une des revendications précédentes,
**caractérisé en ce que** l'appareil est un inhalateur comportant un déclencheur de souffle, dans lequel le déclencheur de souffle déclenche l'acheminement de l'agent propulseur dans l'évaporateur (6).
